# EUROPÄISCHE PATENTSCHRIFT

(11) **EP 3 580 540 B1**
(45) Veröffentlichungstag und Bekanntmachung des Hinweises auf die Patenterteilung: **20.04.2022**
(21) Anmeldenummer: 17826199.6
(22) Anmeldetag: 18.12.2017
(51) Int. Cl.: G01L 5/1627, B25J 9/00, B25J 13/08, G01L 1/22, A61B 90/00

(54) **SENSORANORDNUNG ZUR KRAFT- ODER DREHMOMENTMESSUNG UND EIN VERFAHREN ZUR HERSTELLUNG DERSELBEN**
SENSOR ARRANGEMENT FOR FORCE OR TORQUE MEASUREMENT, AND A METHOD FOR THE PRODUCTION THEREOF
DISPOSITIF CAPTEUR DE MESURE DE FORCE OU DE COUPLE ET SON PROCÉDÉ DE FABRICATION

(30) Priorität: 07.02.2017 DE 102017102343
(43) Veröffentlichungstag der Anmeldung: 18.12.2019
(73) Patentinhaber: WITTENSTEIN SE, 97999 Igersheim (DE)
(72) Erfinder: MATICH, Sebastian, 64319 Pfungstadt (DE); ALBRECHT, Walter, 64285 Darmstadt (DE)
(74) Vertreter: LifeTech IP Spies & Behrndt Patentanwälte PartG mbB
(86) Internationale Anmeldenummer: PCT/EP2017/083320
(87) Internationale Veröffentlichungsnummer: WO 2018/145800

(56) Entgegenhaltungen:
- CN-A- 104 858 892
- DE-A1-102015 003 919
- KR-A- 20120 069 851
- US-A- 5 063 788

## Beschreibung

Die vorliegende Erfindung bezieht sich auf eine Sensoranordnung zur Messung von zumindest einer Komponente einer Kraft oder eines Drehmomentes und ein Verfahren zur Herstellung derselben und insbesondere auf einen in Rolltechnik gefertigten miniaturisierten Kraft-Momentensensor mit einer Hexapodstruktur.

### Hintergrund

Kraft oder Drehmomentsensoren - insbesondere in miniaturisierter Form - können beispielsweise in Telemanipulatoren für eine minimalinvasive Chirurgie zum Einsatz kommen. Solche Telemanipulatoren bestehen aus einer Konsole, über welche beispielsweise der Chirurg zwei im Körperinneren befindliche Manipulationsarme steuern kann. An den Enden der Manipulatoren befinden sich Werkzeuge (Endeffektoren), wie beispielsweise Greifer oder Scheren, die mit dem Gewebe interagieren.

Ein Nachteil von derartigen Telemanipulatoren besteht in dem Verlust der haptischen Wahrnehmung. Um trotzdem die im Körper wirkenden Kräfte dem Chirurgen darstellen zu können, werden die Kontaktkräfte zwischen den Manipulationsarmen und dem Gewebe gemessen. Um einen dabei entstehenden Messfehler möglichst klein zu halten und die Signalgüte zu verbessern, sollten die hierfür genutzten Kraft-Momentensensoren möglichst nahe an dem Endeffektor in dem Manipulationsarm integriert werden. Aufgrund der beengten Platzverhältnisse im Körper sind hierfür jedoch besonders kleine Sensoren erforderlich. Beispielsweise sind Sensoren wünschenswert, die einen Durchmesser von weniger als 10 mm aufweisen und nicht länger als 15 mm sind. Außerdem ist es für solche Sensoren wünschenswert, diese mit einfachen Mitteln in hoher Stückzahl preisgünstig herzustellen.

Bekannte Sensoren, die sowohl Kräfte in den drei Raumrichtungen als auch Drehmomente um die drei Raumrichtungen messen können, basieren beispielsweise auf einem Verformungskörper mit einer Hexapodstruktur, die auch als Stewart-Gough-Plattform bekannt ist. Der Verformungskörper deformiert sich bei einer Krafteinwirkung und diese Deformationen können durch Dehnungsmesselemente erfasst werden. Eine solche Hexapodstruktur ist beispielsweise in der US 3,295,224 A, in der DE 10 2007 017 862 A1, in der US 2011/0314935 A1 oder in der EP 2,260,279 A2 offenbart. Ein Nachteil dieser bekannten Sensoren besteht darin, dass sie einen aufwendig herzustellenden dreidimensionalen Verformungskörper aufweisen, auf dem Dehnungsmesselemente aufgebracht werden. Aufgrund deren monolithischer Bauweise ist die Positionierung, Ausrichtung bzw. das Aufkleben und Andrücken der Dehnungsmesselemente in manuellen Arbeitsschritten auszuführen, die einen hohen Aufwand erfordern. Insbesondere ist das Positionieren der Dehnungsmessstreifen aufgrund der dort gewählten Geometrie bzw. der gewünschten kleinen Größe nur schwer möglich, so dass dieser Prozess nicht oder kaum automatisierbar ist. Das Dokument US 5 063 788 beschreibt ebenfalls, eine Sensoranordnung zur Messung drei Komponenten einer Kraft und drei Komponenten von Drehmoment und ein Verfahren zur Herstellung eines solchen Sensors.

Daher besteht ein Bedarf nach alternativen Sensoranordnungen und Konzepten, die für die genannten Anwendungen einsetzbar sind und zumindest einen Teil der obengenannten Probleme überwinden.

### Zusammenfassung

Zumindest ein Teil der obengenannten Probleme wird durch eine Sensoranordnung nach Anspruch 1, eine Verwendung nach Anspruch 11 und ein Verfahren nach Anspruch 12 gelöst. Die abhängigen Ansprüche definieren weitere vorteilhafte Ausführungsformen für die unabhängigen Ansprüche.

Die vorliegende Erfindung betrifft eine Sensoranordnung zur Messung von zumindest einer Komponente einer Kraft oder eines Drehmomentes. Die Sensoranordnung umfasst die folgenden Merkmale: eine erste Kontaktstruktur und eine zweite Kontaktstruktur, zwischen denen die zumindest eine Komponente der Kraft oder des Drehmomentes zu messen ist. Die Sensoranordnung umfasst weiter mehrere Sensorelemente, die jeweils über ein erstes Gelenk mit der ersten Kontaktstruktur und über ein zweites Gelenk mit der zweiten Kontaktstruktur verbunden und ausgebildet sind, die Komponente der Kraft oder des Drehmomentes zwischen der ersten Kontaktstruktur und der zweiten Kontaktstruktur zu messen. Die erste Kontaktstruktur, die zweite Kontaktstruktur und die mehreren Sensorelemente bilden eine aufgerollte Sensorstruktur, die sich mantelförmig oder spiralförmig entlang einer Oberfläche der Sensoranordnung erstreckt.

Die Begriffe "Kontaktstruktur" oder "Sensorstruktur" oder andere Strukturen sollen breit ausgelegt werden und nicht notwendigerweise sich auf einen Verbund von Teilen beziehen. Vielmehr soll der Aufbau offengelassen werden und insbesondere auch einteilig ausgebildete Teile oder Elemente umfassen. Unter einer Kontaktstruktur soll alles umfasst sein, was zum Kontaktieren (mechanisch oder auch elektrisch) ausgebildet ist. Beispielsweise können die definierten Kontaktstrukturen dazu ausgebildet sein, um Mittel für einen Krafteintrag oder einen Kraftaustrag bereitzustellen. Unter dem Begriff "mantelförmig " im Zusammenhang mit der Sensorstruktur soll verstanden werden, dass die Sensorstruktur nicht monolithisch ist, sondern entlang einer Naht zusammengefügt wurde.

Die mehreren Sensorelemente können beispielhaft sechs Sensorelemente aufweisen, die eine hexagonale Struktur definieren und derart relativ zu der ersten Kontaktstruktur und der zweiten Kontaktstruktur geneigt angeordnet sind, dass dadurch drei verschiedene Kraftkomponenten und/oder drei verschiedene Drehmomentkomponenten unabhängig voneinander messbar sind. Die hexagonale Struktur kann insbesondere eine Hexapodstruktur sein. Optional sind die mehreren Sensorelemente als eine Tripod-, Schnecken- oder Wabenstruktur angeordnet.

Optional können die mehreren Sensorelemente jeweils eine Brückenstruktur mit einem abgedünnten Abschnitt und zumindest einem Dehnungsmesselement auf dem abgedünnten Abschnitt umfassen, um eine Dehnung des abgedünnten Abschnittes als Folge der Einwirkung der Kraft oder des Drehmomentes zu messen. Dehnungsmesselement können Dehnungsmessstreifen sein, aber auch ein oder mehrere Piezoelemente umfassen und beispielhaft an einem Innenbereich nach dem Aufrollen angeordnet sein. Die Piezoelemente können einen oder mehrere Metallfilme umfassen, sie können aber auch als Silizium-Messelemente (piezoresistiver Messeffekt) ausgebildet sein. Zu den vorhandenen Dehnmesselemente können optional zusätzliche Piezoelemente (Aktoren) seitlich daneben angeordnet werden. Die Aktoren können genutzt werden, um die Struktur in hochfrequente Schwingungen zu versetzen, wodurch beispielsweise Hysterese-Effekt minimiert werden können. Dies hätte den Vorteil, dass auch bei geringer Materialgüte, sehr genaue Messungen ermöglicht werden. Optional können die Dehnungsmesselemente ebenfalls Stauchungen (d.h. negative Dehnungen) messen.

Die Brückenstruktur kann beispielsweise ein U-förmiges Querschnittsprofil mit zwei gegenüberliegenden Abschnitten aufweisen, zwischen denen eine Ausnehmung ausgebildet ist und die mit einem Verbindungsabschnitt als abgedünnten Abschnitt überbrückt sind. Optional kann die erste Kontaktstruktur und die zweite Kontaktstruktur an den zwei gegenüberliegenden Abschnitten koppeln und das Dehnungsmesselement kann auf dem Verbindungsabschnitt gebildet sein. Damit wird es möglich, bei der Messung der Komponente der Kraft oder des Drehmomentes einen Hebel auf den Verbindungsabschnitt auszuüben und so eine Dehnungsverstärkung zu erreichen.

Optional kann das erste Gelenk und/oder das zweite Gelenk jeweils ein Festkörpergelenk sein, wobei das Festkörpergelenk eine verringerte, insbesondere quadratische, Querschnittsfläche aufweist. Der quadratische Querschnitt ist nicht zwingend erforderlich (er lässt sich aber leicht durch Schneiden oder Fräsen erzeugen). Mit Blick auf Dehnungsüberhöhungen an den Kanten wäre eine runde Querschnittsfläche einfacher. Diese Querschnittsfläche kann beispielsweise senkrecht zu einer Verbindungslinie zwischen der ersten und zweiten Kontaktstruktur definiert werden. Die konkrete Wahl für die Querschnittsverjüngung stellt einen Kompromiss beispielsweise zwischen einer zuverlässigen Kopplung der gegenüberliegenden Abschnitte (und somit einem ausreichenden Halt) und der leichten Verformbarkeit dar.

Optional umfasst die Sensoranordnung weiter einen ersten Deckel und/oder einen zweiten Deckel, wobei der erste Deckel an der ersten Kontaktstruktur befestigt ist und der zweite Deckel an der zweiten Kontaktstruktur befestigt ist.

Die Deckel können insbesondere Mittel zur Krafteinleitung und Kraftausleitung umfassen. Diese Mittel umfassen beispielsweise Halteelemente oder Gewinde, um eine Schraubverbindung herzustellen.

Die Sensoranordnung kann beispielsweise zur Kraftübertragung auf ein Werkzeug genutzt werden. Dazu können der erste Deckel und der zweite Deckel eine axiale Achse definieren, um die die mantelförmige Sensorstruktur herum angeordnet ist. Außerdem können der erste Deckel und der zweite Deckel jeweils eine Öffnung aufweisen, durch die die axiale Achse verläuft. Die axiale Achse kann auch durch eine entsprechende Anordnung der Mittel zur Krafteinleitung und zur Kraftausleitung definiert sein. Die aufgerollte Sensorstruktur kann außerdem einen Innenraum definieren, um ein Verlegen von optischen und/oder elektrischen Leitungen und/oder von Elementen zur Bedienung des Werkzeuges entlang der axialen Achse durch die Sensoranordnung hindurch zu ermöglichen. Es können auch integrierte Schaltungen zur Auswertung der Messelemente in der Sensorstruktur liegen, um so die Anzahl der Kabel zu reduzieren. Optional umfassen die erste Kontaktstruktur und die zweite Kontaktstruktur jeweils eine Vielzahl von Segmenten. Außerdem kann jeweils ein Sensorelement zwischen zwei entsprechenden Segmenten ausgebildet sein. Auf diese Weise wird eine Vielzahl von Sensorabschnitten gebildet. Optional umfassen die Segmente jeweils einen Zapfen, der sich von dem Sensormodul weg erstreckt. Der erste Deckel und der zweite Deckel können jeweils eine Vielzahl von Nuten aufweisen, die derart angeordnet sind, dass die Zapfen der ersten und zweiten Kontaktstruktur in die Nuten einführbar sind. Optional können die Zapfen durch ein Verkleben und/oder Verlöten und/oder Verschweißen (z.B. Widerstandsschweißen) fest mit den Deckeln verbunden werden. Weitere Techniken umfassen das Fügen durch Umformen, wobei z.B. crimpen, nieten oder biegen sinnvoll eingesetzt werden können. Dies hätte den Vorteil, dass nur ein geringer oder kein Wärmeeintrag stattfindet. Der Deckel erfüllt außerdem die Funktion, die Kontaktstrukturen zu verbinden.

Optional umfasst die Sensoranordnung zusätzliche Sensorelemente auf den Segmenten der ersten Kontaktstruktur und/oder auf den Segmenten der zweiten Kontaktstruktur. Beispielsweise können jeweils ein Sensorelement mit einem der zusätzlichen Sensorelemente zu einer Halbbrückenschaltung verschaltet werden. Durch weitere zusätzliche Sensorelemente kann ebenfalls eine Vollbrückenverschaltung von Sensorelementen erreicht werden. Die zusätzlichen Sensorelemente werden beispielsweise derart an den Segmenten fixiert, dass sie eine Vergleichsmessung erlauben, um die Genauigkeit zu erhöhen. Zum Beispiel können sie auf einen sich nicht dehnenden Abschnitt aufgebracht werden und/oder in einer anderen Richtung sensitiv sein.

Ausführungsbeispiele der vorliegenden Erfindung beziehen sich auch auf eine Verwendung der zuvor beschriebenen Sensoranordnung als einen miniaturisierten Kraft-Momentensensor für die minimalinvasive Chirurgie.

Die vorliegende Erfindung bezieht sich auch auf ein Verfahren zur Herstellung eines Sensors zur Messung von zumindest einer Kraft und eines Drehmomentes. Das Verfahren umfasst die Schritte:
- Bereitstellen einer planaren Sensorstruktur mit einer ersten Kontaktstruktur und einer zweiten Kontaktstruktur mit dazwischen gelenkförmig verbundenen mehreren Sensorelementen; und
- Aufrollen der planaren Sensorstruktur, so dass die erste Kontaktstruktur und die zweite Kontaktstruktur und die mehreren Sensorelemente sich mantelförmig oder spiralförmig um eine axiale Achse herum erstrecken.

Optional kann das Bereitstellen der planaren Sensorstruktur folgende Schritte umfassen:
- Bereitstellen eines biegsamen Grundkörpers;
- Strukturieren des biegsamen Grundkörpers, um die erste Kontaktstruktur und die zweite Kontaktstruktur zu bilden, die durch Brückenelemente miteinander verbunden sind; und
- Ausbilden von zumindest jeweils einem Dehnungsmesselement auf den Brückenelementen.

Die Strukturierung kann beispielsweise zumindest einen der folgenden Arbeitsgänge umfassen: ein Fräsen, ein Schneiden, ein Schleifen, eine Laserbearbeitung (wie z.B. Lasersintern oder Laserschneiden), ein Ätzen, Schneiden (Stanzen), Prägen, 3D-Druck.

Die Herstellung kann durch einen zweistufigen Prozess, der für eine günstige Massenfertigung geeignet ist, erfolgen, der folgende Schritte umfasst:
1. Ein Blech wird auf der Fräse Bearbeitet, wobei Strukturen, die in die Ebene steigen, gefräst werden.
2. Die so vorbearbeitete Platte kommt auf den Laser und die Strukturen werden ausgeschnitten.

Toleranzkritische Strukturen können präzise auf der Fräse herausgearbeitet werden. Außerdem lässt sich die Fräszeit massiv verkürzen, wenn die Struktur ausgeschnitten und nicht ausgefräst wird. Außerdem kann optional ein Spannungsfreiglühen der planaren Sensorstruktur erfolgen, um vorhandene Spannungen im Material zu beseitigen. Damit kann eine deutliche Verbesserung der Messung durch Dehnungsmessstreifen erreicht werden.

Optional umfasst der Schritt des Aufrollens:
- ein Einführen der planaren Sensorstruktur in eine Aufrollvorrichtung, die eine Führung und einen abgewinkelten Abschnitt umfasst, wobei die Führung ausgebildet ist, um die planare Sensorstruktur aufzunehmen, und
- ein Bewegen der planaren Sensorstruktur hin zu dem abgewinkelten Abschnitt, wodurch die planare Sensorstruktur abschnittsweise verbogen und die mantelförmige Struktur erzeugt wird.

Das Verfahren kann optional ein Aufsetzen eines ersten Deckels und eines zweiten Deckels auf gegenüberliegenden Seiten der aufgerollten Sensorstruktur umfassen. Außerdem kann die aufgerollte Sensorstruktur in einer zylinder- oder prismenförmigen Montagehilfe eingeführt werden. Schließlich kann unter Ausübung eines Druckes auf die gegenüberliegenden Deckel erreicht werden, dass die mantelförmig angeordnete Sensorstruktur sich axialsymmetrisch ausrichtet.

Ausführungsbeispielen der vorliegenden Erfindung bieten die folgenden Vorteile:
Zunächst ist die Sensoranordnung gemäß der vorliegenden Erfindung problemlos in miniaturisierter Form herstellbar. Die Probleme der bekannten Sensoren hinsichtlich der Fertigung des Verformungskörpers und des anschließenden Applizierens (z.B. Aufkleben) von Dehnungsmesselementen auf den Verformungskörper und die Kontaktierung und Verkabelung der applizierten Dehnungsmesselemente werden durch Ausführungsbeispiele dadurch überwunden, dass die Sensoranordnung planar hergestellt wird und durch einen Aufrollprozess die räumliche Gestalt des Verformungskörpers erreicht wird.

Im Gegensatz zu den konventionellen Anordnungen, bei denen wegen ihrer monolithischen Bauweise die Innenseiten des Zylinders nicht erreichbar ist und somit auch das Applizieren der Messelemente auf der Innenseite schwierig oder unmöglich ist, stellt dies bei Ausführungsbeispielen - auch bei einem hohen Miniaturisierungsgrad - kein Problem dar.

Als Dehnungsmesselemente können einfache Folien-Dehnungsmesselemente oder Silizium-Dehnungsmesselemente oder auch eine Folie mit mehreren Messelementen, die in einem Arbeitsschritt appliziert wird, genutzt werden. Dadurch wird eine kostengünstige Fertigung ermöglicht. Die Kräfte können bei Ausführungsbeispielen in allen drei Raumrichtungen x, y und z und die Drehmomente um diese Achsen können unabhängig voneinander gemessen werden. Wegen der planaren Fertigung ist es ebenfalls möglich, Dünnschicht- und Dickschichttechniken zur Herstellung der Sensoren zu nutzen. Diese massentauglichen Techniken funktionieren nicht für die eingangs erwähnten monolithischen Strukturen. Ausführungsbeispiele erlauben es ebenso, Dehnmesselemente aufzudrucken oder mittels Paste und Laser zu strukturieren.

### Kurzbeschreibung der Figuren

Die Ausführungsbeispiele der vorliegenden Erfindung werden besser verstanden von der folgenden detaillierten Beschreibung und den beiliegenden Zeichnungen der unterschiedlichen Ausführungsbeispiele, die jedoch nicht so verstanden werden sollten, dass sie die Offenbarung auf die spezifischen Ausführungsformen einschränkt, sondern lediglich der Erklärung und dem Verständnis dienen.
- Fig. 1: zeigt eine Sensoranordnung zur Messung von zumindest einer Komponente einer Kraft oder eines Drehmomentes gemäß einem Ausführungsbeispiel der vorliegenden Erfindung.
- Fig. 2: zeigt schematisch eine Sensorstruktur gemäß einem Ausführungsbeispiel in der abgerollten Form.
- Fig. 3: zeigt schematisch die aufgerollte Sensorstruktur aus der Fig. 2.
- Fig. 4: zeigt eine detaillierte Ansicht der abgerollten Sensorstruktur gemäß weiterer Ausführungsbeispiele.
- Fig. 5: zeigt eine vergrößerte Darstellung der in der Fig. 4 unten gezeigten brückenförmigen Struktur.
- Fig. 6: zeigt die in der Fig. 4 dargestellte planare Sensorstruktur in dem aufgerollten Zustand.
- Fig. 7A, 7B: zeigen eine planare Sensorstruktur gemäß einem weiteren Ausführungsbeispiel der vorliegenden Erfindung.
- Fig. 8A, 8B: zeigen eine Seitenansicht und eine Raumansicht der aufgerollten Sensorstruktur.
- Fig. 9: zeigt eine Vorrichtung zum Aufrollen der Sensoranordnung gemäß Ausführungsbeispielen.
- Fig. 10: zeigt eine Montagehilfe zum Montieren zum der Sensoranordnung gemäß Ausführungsbeispielen.

### Detaillierte Beschreibung

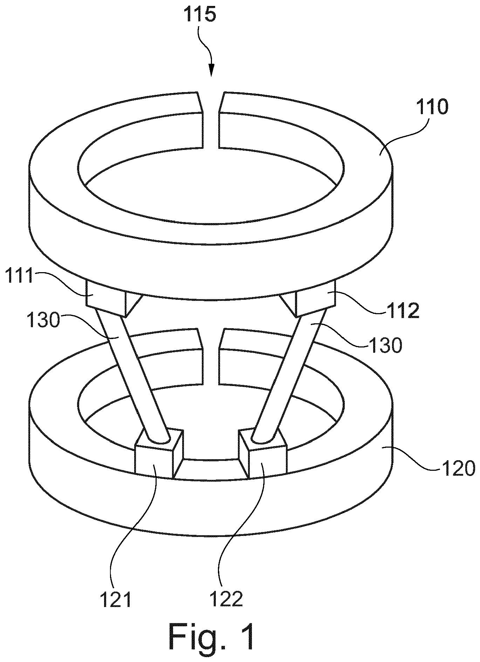

**Fig. 1** zeigt eine Sensoranordnung zur Messung von zumindest einer Komponente einer Kraft oder eines Drehmomentes gemäß einem Ausführungsbeispiel der vorliegenden Erfindung. Die Sensoranordnung umfasst eine erste Kontaktstruktur 110 und eine zweite Kontaktstruktur 120, zwischen denen zumindest eine Komponente der Kraft oder des Drehmomentes wirkt. Außerdem umfasst die Sensoranordnung mehrere Sensorelemente 130, die jeweils über ein erstes Gelenk 111, 112 mit der ersten Kontaktstruktur 110 und über ein zweites Gelenk 121, 122 mit der zweiten Kontaktstruktur 120 verbunden sind. Die Sensorelemente 130 sind ausgebildet, um die Komponente der Kraft oder des Drehmomentes zwischen der ersten Kontaktstruktur 110 und der zweiten Kontaktstruktur 120 zu messen. Dies kann beispielsweise durch ein Messen von Dehnungen erfolgen (z.B. unter Nutzung von Dehnungsmessstreifen). Denkbar ist aber auch die Nutzung von Piezoelemente oder andere Messsensoren oder Sensorprinzipien (wie z.B. optische Verfahren), die zur Kraftmessung geeignet sind. Wie bereits dargelegt, können die Piezoelemente einen oder mehrere Metallfilme umfassen oder als Silizium-Messelemente (piezoresistiver Messeffekt) ausgebildet sein. Zu den vorhandenen Dehnmesselementen können optional zusätzliche Piezoelemente (Aktoren) seitlich daneben angeordnet werden. Die Aktoren können genutzt werden, um die Struktur in hochfrequente Schwingungen zu versetzen, wodurch beispielsweise Hysterese-Effekt minimiert werden können. Dies hätte den Vorteil, dass auch bei geringer Materialgüte, sehr genaue Messungen ermöglicht werden.

Gemäß der vorliegenden Erfindung bilden die erste Kontaktstruktur 110, die zweite Kontaktstruktur 120 und die mehreren Sensorelemente 130 eine aufgerollte Struktur, die sich mantelförmig entlang einer Oberfläche der Sensoranordnung erstreckt. Die mantelförmig aufgerollte Struktur mit der ersten Kontaktstruktur 110 und der zweiten Kontaktstruktur berühren einander entlang einer Verbindungsstelle 115. Optional kann dort auch ein Spalt ausgebildet sein, der mit einem Klebstoff, eine Lötnaht oder anderweitig verschlossen sein kann. Der Spalt 115 ist dann beispielsweise durch das unterschiedliche Material, das zum Verschließen genutzt wird, erkennbar.

In dem Ausführungsbeispiel der Fig. 1 sind beispielhaft nur zwei Sensorelemente 130 gezeigt. Vorteilhafterweise sind zumindest drei Sensorelemente ausgebildet. Dies schafft einerseits eine ausreichende Stabilität beim Halten der ersten und zweiten Kontaktstruktur 110, 120, ermöglicht aber außerdem drei unabhängige Messungen für drei der sechs Komponenten (drei Translationskräfte und drei Drehmomentkräfte). Die vorliegende Erfindung soll jedoch nicht auf eine bestimmte Anzahl von Sensorelementen 130 eingeschränkt werden - je nach Anwendung können mehr oder weniger Sensoren vorhanden sein.

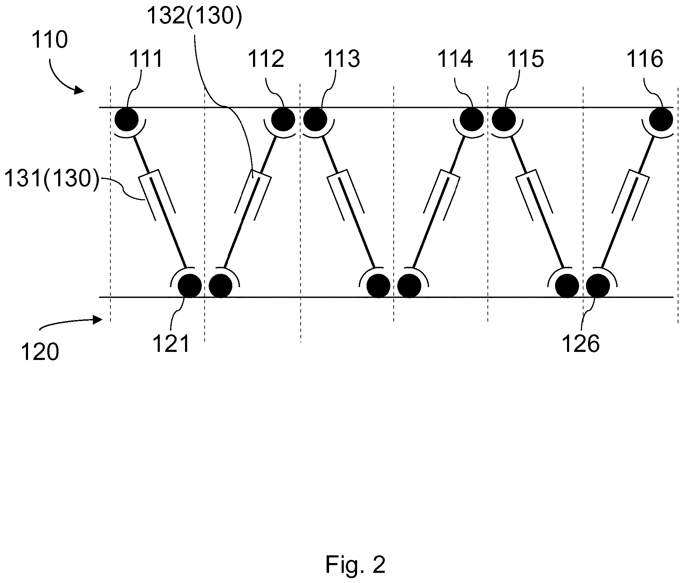

Fig. **2** zeigt die Sensoranordnung in der abgerollten Form mit insgesamt sechs Sensorelementen 131, 132, ... 136. Die beispielhaft gezeigten sechs Sensorelemente 131 ... 136 sind über Gelenke 111, 112, ... 121, 122, ... mit der ersten Kontaktstruktur 110 bzw. der zweiten Kontaktstruktur 120 verbunden. So ist das erste Sensorelement 131 mit einem ersten Gelenk 111 mit der ersten Kontaktstruktur 110 und mit einem zweiten Gelenk 121 mit der zweiten Kontaktstruktur 120 verbunden. In der gleichen Weise ist das zweite Sensorelement 132 wiederum über ein entsprechend erstes Gelenk 112 mit der ersten Kontaktstruktur 110 und ein entsprechend zweites Gelenk 122 mit der zweiten Kontaktstruktur 120 verbunden. In ähnlicher Weise sind alle anderen Sensorelemente beidseitig über Gelenke mit der ersten Kontaktstruktur 110 und der zweiten Kontaktstruktur 120 verbunden.

Die ersten Gelenke 111, 112, ... 116 und die zweiten Gelenke 121, 122, ..., 126 können beispielsweise Festkörpergelenke sein, die ein relatives Verkippen der Sensorelemente 131, ... 136 relativ zu der ersten Kontaktstruktur 110 bzw. der zweiten Kontaktstruktur 120 erlauben. Die Sensorelemente 131, ... 136 können außerdem relativ zu der ersten Kontaktstruktur 110 und der zweiten Kontaktstruktur 120 geneigt angeordnet sein. Dies ist zwar nicht zwingend erforderlich, bietet jedoch den Vorteil, dass die Sensitivität und die Stabilität der Sensoranordnung verbessert wird. Es hat sich gezeigt, dass insbesondere bei einem Neigungswinkel α zwischen 30° und 60° (oder bei ca. 35° oder bei ca. 55° oder zwischen diesen Werten) relativ zu einer vertikalen Achse (senkrechte Verbindungachse zwischen der ersten und zweiten Kontaktstruktur 110, 120) eine sehr hohe Sensitivität erreichbar ist.

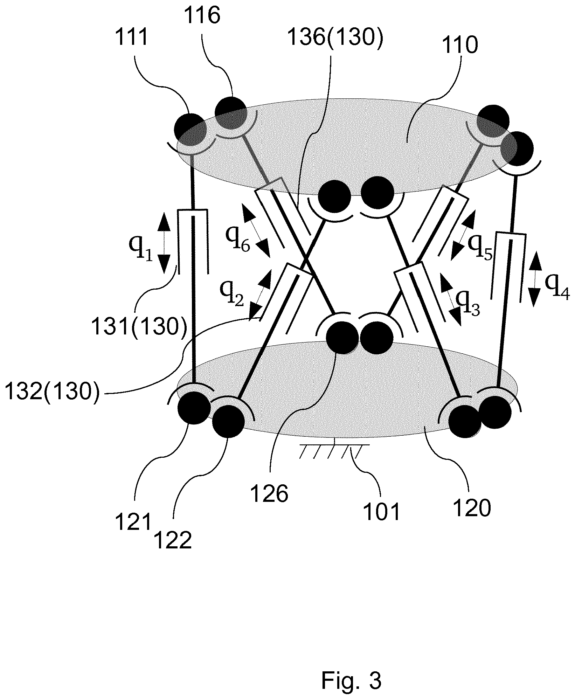

**Fig. 3** zeigt schematisch die aufgerollte Sensorstruktur, nachdem die planar angeordnete Sensorstruktur aus der Fig. 2 zylinderförmig oder prismenförmig aufgerollt wurde. In dem gezeigten Ausführungsbeispiel ist wiederum die erste Kontaktstruktur 110 oben angeordnet und die zweite Kontaktstruktur 120 unten angeordnet. Eine Verkippung oder Bewegung der ersten Kontaktstruktur 110 relativ zur zweiten Kontaktstruktur 120 führt dazu, dass zumindest einige der Sensorelemente 131, ... 136 ihre Länge ändern. Diese Längenänderung ist durch die Variablen q1, q2, ... q6 für die einzelnen Sensorelemente 131, 132, ...136 dargestellt. Außerdem führt die Verkippung oder Bewegung der ersten Kontaktstruktur 110 relativ zur zweiten Kontaktstruktur 120 dazu, dass sich die Sensorelemente 130 relativ zur ersten Kontaktstruktur 110 und der zweiten Kontaktstruktur 120 verkippen, wozu die Gelenkverbindungen 111, ... 116 und 121,... 126 ausgebildet sind.

Die in der Fig. 3 gezeigte Darstellung ist eine vereinfachte Darstellungsform, bei welcher die streifenförmig angeordneten Sensorelemente 131, ... 136 entlang des Umfanges der zylinderförmig oder prismenförmig aufgerollten Sensorstruktur idealerweise nicht biegsam zueinander sind und nur ihre Länge entsprechend ändern können, um ein entsprechendes elektrisches Signal zu liefern. Dazu kann beispielsweise die zweite Sensoranordnung 120 mit einer Masse 101 verbunden werden und entsprechende Signale können über die erste Kontaktstruktur 110 erfasst und ausgegeben werden. Die zweite Kontaktstruktur 120 stellt beispielhaft eine Basis und die erste Kontaktstruktur 110 eine Plattform dar. Die erfassten sechs Signale, die eine relative Längenänderung der einzelnen streifenförmig ausgebildeten Sensorelemente 130 darstellen, können ausgewertet und zur Bestimmung der drei möglichen lateralen Kraftrichtungen und der drei möglichen Drehmomentrichtungen genutzt werden.

Fig. 3 zeigt somit eine kinematische Struktur eines Hexapoden (Stewart-Gough-Plattform), deren Abrollen und Segmentierung zu der Darstellung aus der Fig. 2 führt.

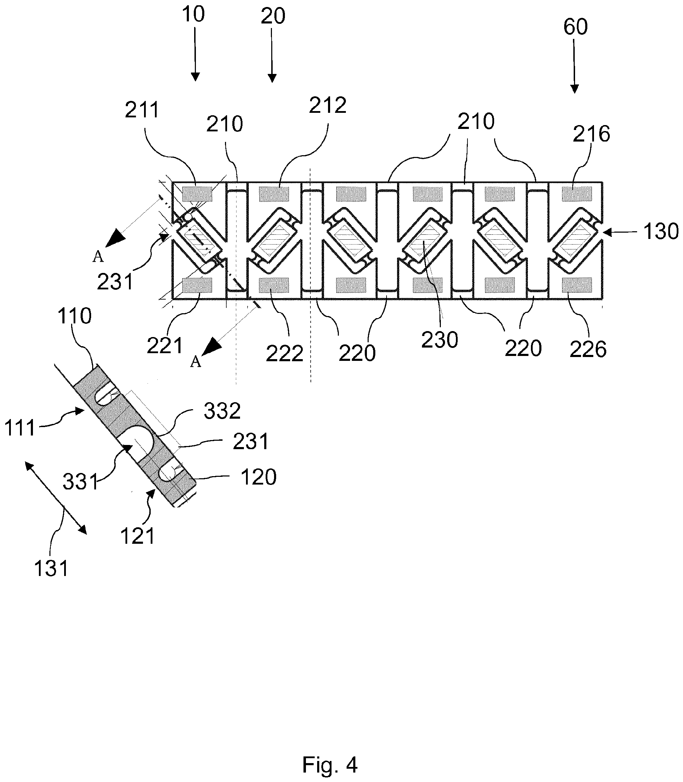

**Fig. 4** zeigt eine detaillierte Ansicht der abgerollten Sensorstruktur, wobei die erste Kontaktstruktur 110 und die zweite Kontaktstruktur 120 zusammen mit den Sensorelementen 130 planar angeordnet sind. In dem Ausführungsbeispiel der Fig. 4 sind insgesamt sechs Sensorabschnitte 10, 20, ... 60 ausgebildet (die Trennung erfolgt, wie durch die gestrichelten Linien gezeigt), wobei jeder Abschnitt ein Segment der ersten Kontaktstruktur 110 und ein Segment der zweiten Kontaktstruktur 120 und ein Sensorelement 130 umfasst. Die sechs Sensorabschnitte 10, 20, ... 60 sind dabei über erste Verbindungsabschnitte 210 und zweite Verbindungsabschnitte 220 miteinander verbunden. Beispielhaft umfasst jedes Sensorelement 130 einen Dehnungsmessstreifen 230.

Die ersten Verbindungsabschnitte 210 verbinden dabei jeweils die entsprechenden Segmente der ersten Kontaktstruktur 110. Die zweiten Verbindungsabschnitte 220 verbinden dabei jeweils die entsprechenden Segmente der zweiten Kontaktstruktur 120. Die ersten Verbindungsabschnitte 210 und die zweiten Verbindungsabschnitte 220 sind beispielsweise verdünnt ausgebildete Abschnitte, so dass es möglich wird, die dargestellte planare Sensorstruktur zylinderoder prismenförmig aufzurollen, so dass der erste Abschnitt 10 mit dem sechsten Sensorabschnitt 60 in Verbindung gelangt oder zumindest in dessen Nähe gebracht wird. Die Verbindungsabschnitte 210, 220 können beispielsweise Filmgelenke sein.

Außerdem umfasst jeder Sensorabschnitt 10, 20, ... 60 jeweils ein Kontaktpad 211 auf der ersten Kontaktstruktur 110 und ein Kontaktpad 221, 222, ... 226 auf der zweiten Kontaktstruktur 120. Die Kontaktpads 211, 212,... 216 und 221, 222, ... 226 sind ausgebildet, um einen elektrischen Kontakt zwischen dem jeweiligen Kontaktpad und dem beispielhaften Dehnungsmesstreifen 230 herzustellen. Die Kontaktpads 211, 221, 212, 222 bieten außerdem Platz für eine Primärelektronik oder eine Kontaktierung (z.B. durch Bonden), um eine Signalerfassung für eine (externe) Auswerteeinheit zu ermöglichen. Die Gelenke 111, 121 zum Halten der Sensorelemente 130 können wiederum Festkörpergelenke oder Kugelgelenke sein.

Außerdem zeigt die Fig. 4 unten links eine Querschnittsansicht entlang der Querschnittslinie A-A, die durch das erste Sensorelement 131 verläuft, das mit dem ersten Festkörpergelenk 111 an der ersten Kontaktstruktur 110 und mit dem zweiten Festkörpergelenk 121 an der zweiten Kontaktstruktur 120 befestigt ist. Die Querschnittsansicht zeigt, dass das erste Festkörpergelenk 111 und das zweite Festkörpergelenk 121 durch abgedünnte Abschnitte gebildet sind, so dass an diesen Abschnitten relative Verbiegungen zwischen der ersten Kontaktstruktur 110 und dem ersten Sensorelement 131 als auch zwischen der zweiten Kontaktstruktur 120 und dem ersten Sensorelement 131 möglich sind.

Außerdem ist das erste Sensorelement 131 brückenförmig ausgebildet, indem in einem zentralen Bereich eine Ausnehmung 331 (oder Hohlraum oder Ausfräsung) gebildet wird, die durch einen abgedünnten Abschnitt 332 als ein Verbindungsabschnitt überbrückt wird, auf dessen Oberfläche beispielhaft der Dehnungsmessstreifen 231 ausgebildet ist.

Die Ausnehmung 331 befindet sich auf einer gleichen Seite der brückenförmigen Struktur wie die abgedünnten Abschnitte, die das erste Festkörpergelenk 111 und das zweite Festkörpergelenk 121 bilden. Diese Gestaltung bietet den folgenden Vorteil. Eine Kraft, die entlang der Schnittlinie A-A wirkt, wird über die Festkörpergelenke 111, 121 an jener Seite eingebracht, wo die Ausnehmung 331 vorhanden ist und folglich nicht direkt weitergeleitet. Wegen der Ausnehmung 331 wird sie umgeleitet und verbiegt den Verbindungsabschnitt 332. Die Brückenstruktur bewegt sich ziehharmonikaförmig auseinander. Diese Verbiegung ist durch den beispielhaften Dehnungsmessstreifen 231 als eine Dehnung (oder auch Stauchung) feststellbar.

Es ist von besonderem Vorteil, wenn die Tiefe der Ausnehmung 331 und die Position des ersten Festkörpergelenks 111 und/oder des zweiten Festkörpergelenks 221 so gewählt werden, dass die Komponente der Kraft oder des Drehmomentes einen Hebel auf den Verbindungsabschnitt 332 ausübt und so zu einer Dehnungsverstärkung (oder Dehnungsüberhöhung) führt. Der Grad der Verstärkung kann z.B. durch Tiefe der Ausnehmung 331 bzw. die Dicke des Verbindungsabschnittes 332 an der dünnsten Stelle flexibel eingestellt werden.

Alle weiteren Abschnitte und Sensorelemente 130 können in der gleichen Form ausgebildet sein. Die einzelnen Sensorelemente 130 in den Sensorabschnitten 20, ... 60 unterscheiden sich lediglich dadurch, dass die relative Ausrichtung zu der ersten Kontaktstruktur 110 und zu der zweiten Kontaktstruktur 120 geändert ist, so dass sie besonders sensitiv für andere Raumrichtungen bzw. Drehrichtungen sind. Beispielsweise können benachbarte Brückenstrukturen der Sensorelemente 130 sich jeweils in einem rechten Winkel (oder zwischen 60° ... 120°) voneinander erstrecken, so dass sie für orthogonal aufeinander wirkende Kräfte besonders sensitiv sind.

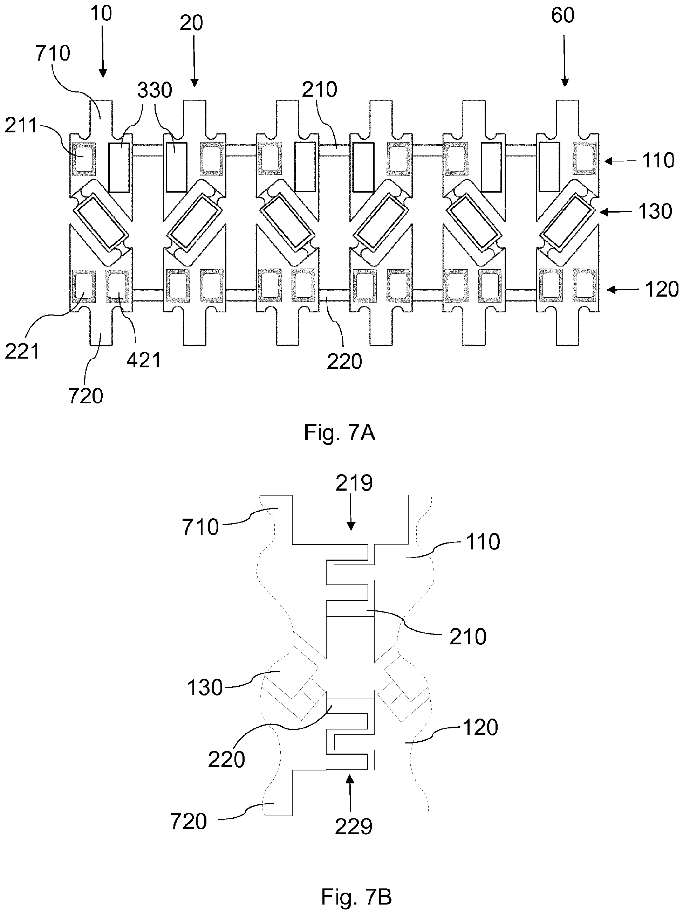

Die aufgebrachten Dehnungsmessstreifen 231 können beispielsweise Foliendehnungsmessstreifen oder Siliziumdehnungsmessstreifen sein, die als eine Viertelbrücke verschaltet werden. Es können auch mehrere Messstreifen genutzt werden, die als eine Halb- oder Vollbrücke miteinander verschaltet werden (siehe auch Fig. 7).

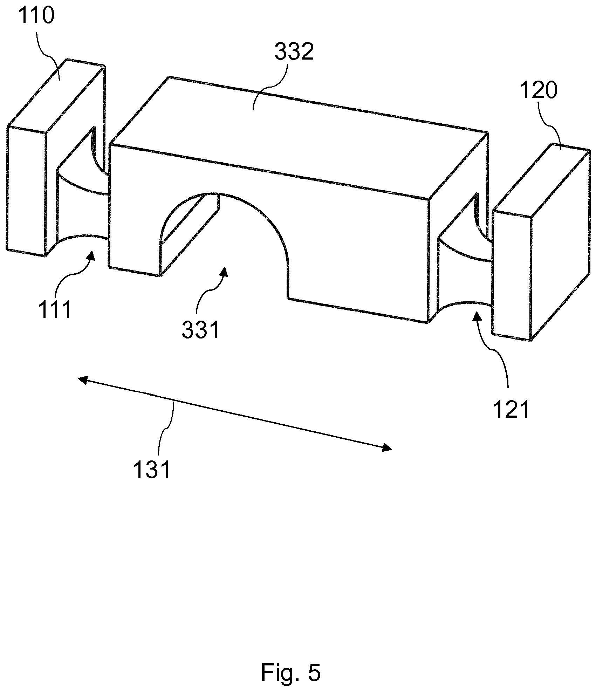

**Fig. 5** zeigt eine vergrößerte Darstellung der in der Fig. 4 unten links gezeigten brückenförmigen Struktur für das erste Sensorelement 131, das zwischen Teilen der ersten Kontaktstruktur 110 und der zweiten Kontaktstruktur 120 dargestellt ist, die mittels dem ersten Festkörpergelenk 111 und dem zweiten Festkörpergelenk 121 fest verbunden sind. Die Geometrie der Festkörpergelenke 111, 121 ist derart gewählt, dass sich die erste Kontaktstruktur 110 relativ zu dem ersten Sensorelement 131 neigen lässt, ohne dass es dabei zu einem Bruch des ersten Festkörpergelenkes 111 kommt. In gleicher Weise ist das zweite Festkörpergelenk 121 ausgebildet ist, um ein relatives Neigen oder Verkippen der zweiten Kontaktstruktur 120 relativ zu dem ersten Sensorelement 131 zu erlauben. Dies kann durch eine entsprechende Materialwahl (z.B. mit einer passenden Duktilität) erreicht werden.

Insbesondere kann aber die Querschnittsfläche senkrecht zur Erstreckung der Brücke deutlich kleiner sein als jene von der Brückenstruktur beidseitig der Ausnehmung 331 (z.B. weniger als 50% oder weniger als 30%). Dies kann beispielsweise über Auskerbungen geschehen, die z.B. mittels Fräsen hergestellt werden können. Dabei ist es insbesondere von Vorteil, wenn diese Querschnittfläche in beiden Raumrichtungen verkleinert wird (z.B. in Form eines Quadrats), um eine Gelenkwirkung in beiden Richtungen zu erreichen.

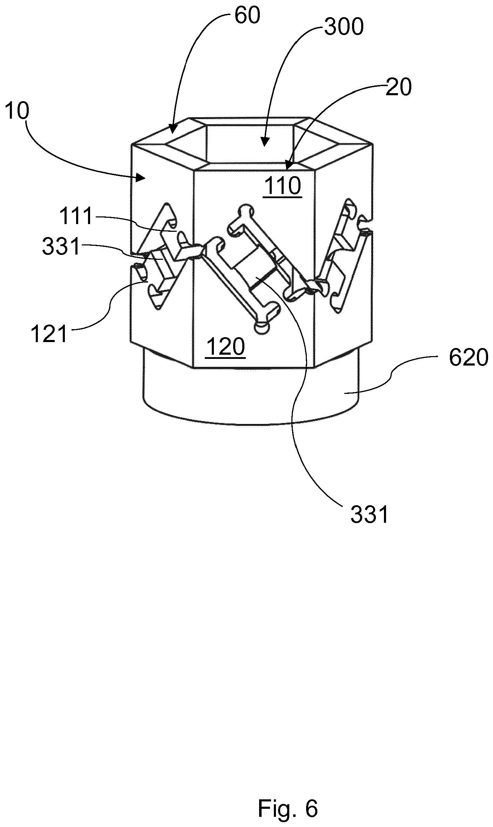

**Fig. 6** zeigt die in der Fig. 4 dargestellte planare Sensorstruktur in dem aufgerollten prismenförmigen Zustand, sodass ein Innenraum 300 gebildet wird. Das Aufrollen kann derart geschehen, dass die überbrückten Ausnehmungen 331, 332, ... der Brückenstrukturen auf einer äußeren Oberfläche (abgewandt vom Innenraum 300) der prismenförmig aufgerollten Sensoranordnung sich befinden. Dadurch wird erreicht, dass die beispielhaften Dehnungsmessstreifen 230 geschützt in dem Innenraum 300 angeordnet sind. Außerdem können die Festkörpergelenke 111, 121, ... auf der gezeigten äußeren Oberfläche bündig mit der ersten Kontaktstruktur 110 und bündig mit der zweiten Kontaktstruktur 120 abschließen. Die entsprechenden Auskerbungen zwischen der ersten Kontaktstruktur 110 und dem Sensorelement 131 bzw. zwischen der zweiten Kontaktstruktur 120 und dem Sensorelement 131 befindet sich und in dem Innenbereich 300 (siehe auch Fig. 5).

Wie auch in den zuvor gezeigten Darstellungen, sind in der aufgerollten Struktur sechs Sensorabschnitte 10, 20, ... 60 miteinander verbunden, so dass sich ein hexagonales Prisma mit einer sechseckförmigen Grundfläche bzw. Querschnittsebene ergibt.

Die Fig. 6 zeigt außerdem beispielhaft einen Deckel 620, der die zweite Kontaktstruktur 120 hält und beispielsweise aufgeklebt oder aufgeschweißt werden kann. Auf der Oberseite entlang der ersten Kontaktstruktur 110 kann ebenfalls ein Deckel ausgebildet werden, der in der Fig. 6 nicht gezeigt ist. Die Deckel auf der Ober- und Unterseite dienen der Krafteinleitung bzw. Kraftausleitung zum Ansteuern der Bedienelemente (z.B. für Werkzeuge oder Endeffektoren, die an den Enden der Manipulatoren vorhanden sein können).

**Fig. 7A** zeigt ein weiteres Ausführungsbeispiel der vorliegenden Erfindung, welches sich von dem in der Fig. 4 gezeigten Ausführungsbeispiel dadurch unterscheidet, dass jeder Sensorabschnitt 10, 20, ... 60 drei Kontaktpads 211, 221, 421 und zusätzlich zu dem bereits vorhandenen Sensorelement 130 zumindest ein zusätzliches Sensorelement 330 aufweist. Das zumindest eine zusätzliche Sensorelement 330 kann in einem Bereich der Kontaktstrukturen 110, 120 gebildet werden, der sich bei der Kraft- oder Drehmomentmessung kaum oder nicht deformieren wird, sodass es für eine Vergleichsmessung genutzt werden kann. Beispielsweise kann mittels des zumindest einen zusätzlichen Sensorelementes 330 eine Halbbrückenstruktur erzeugt werden, die über die drei Kontaktpads 211, 221, 421 jeweils kontaktiert wird. Die drei Kontaktpads 211, 221, 421 sind dazu entsprechend mit den jeweiligen Sensorelementen 130 und zusätzlichen Sensorelementen 330 elektrisch verbunden. Beispielsweise sind auf den Segmenten der ersten Kontaktstruktur 110 in jedem Abschnitt 10, 20, ... jeweils ein Kontaktpad 211 und ein zusätzliches Sensorelement 330 und auf den Segmenten der zweiten Kontaktstruktur 120 jeweils zwei Kontaktpads 221, 421 ausgebildet. Die Verteilung kann jedoch auch anders gewählt werden.

Außerdem umfasst jedes Segment der ersten Kontaktstruktur 110 und jedes Segment der zweiten Kontaktstruktur 120 in dem gezeigten Ausführungsbeispiel jeweils einen zapfenförmig ausgebildeten Abschnitt. So umfasst der erste Abschnitt 10 einen ersten Zapfen 710 an der erste Kontaktstruktur 110 und einen zweiten Zapfen 720 an der zweiten Kontaktstruktur 120. Die anderen Abschnitte 20, ... , 60 können in gleicher Weise ausgebildet werden.

Die Fig. 7B zeigt eine weitere Ausführungsform der vorliegenden Erfindung, die sich von dem Ausführungsbeispiel der Fig. 7A dadurch unterscheidet, dass die ersten und zweiten Verbindungsabschnitte 210, 220 näher an den Sensorelementen 130 angeordnet sind. Außerdem ist zusätzlich eine erste Kammstruktur 219 zwischen den Segmenten der ersten Kontaktstruktur 110 und eine zweite Kammstruktur 229 zwischen den Segmenten der zweiten Kontaktstruktur 120 ausgebildet. Die Kammstrukturen 219, 229 sind durch ineinandergreifende Kämme definiert, die die Haftoberfläche zwischen der ersten Kontaktstruktur 110 und der zweiten Kontaktstruktur 120 vergrößert.

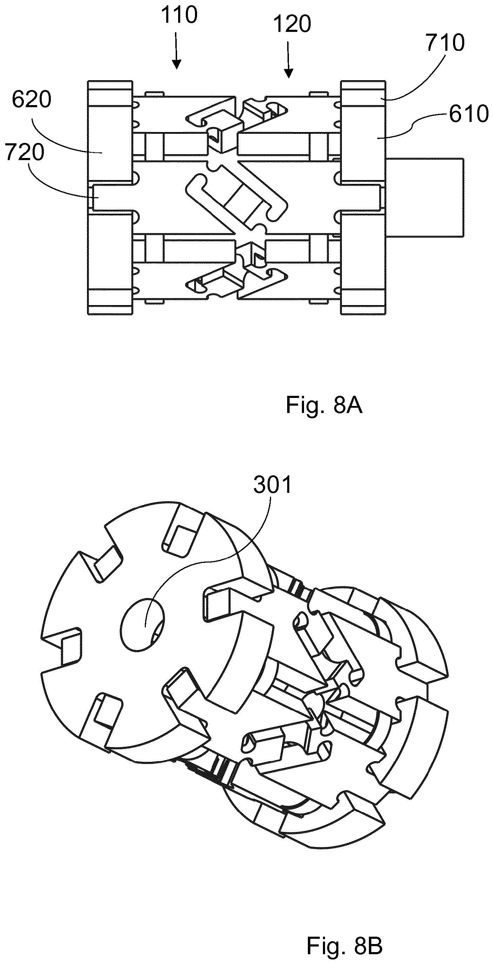

Zum Beispiel sind vor dem Aufrollen der planaren Sensorstruktur die ersten Kontaktstruktur 110 und die zweiten Kontaktstruktur 120 entlang der ersten und zweiten Kammstruktur 219, 229 nicht miteinander verbunden (sondern nur über die ersten und zweiten Verbindungselemente 210, 220). Nach dem Aufrollen können die erste und zweite Kammstrukturen 219, 229 genutzt werden, um die erste und die zweite Kontaktstruktur 110, 120 miteinander zu verbinden (z.B. über ein Kleben, Schmelzen, Löten, Laser-Behandlung, etc.). Die vergrößerte Haftoberfläche schafft somit eine zuverlässige Verbindung. Dies bietet den Vorteil, dass die Deckel zwingenderweise nicht erforderlich sind (siehe Fig. 8A und 8B). Auch die Zapfen 710, 720 sind in dieser Ausführungsform optional. Sie können aber genutzt werden, um eine Ankopplung der Sensoranordnung an anderen Elementen zu erreichen.

Die Sensorelemente 130 sind in der gleichen Art ausgebildet, wie sie zuvor beschrieben wurde, so dass eine wiederholte Beschreibung nicht erforderlich ist.

**Fig. 8A** zeigt Seitenansicht und **Fig. 8B** eine Raumansicht der aufgerollten Sensorstruktur, die mit einem ersten Deckel 610 an der ersten Kontaktstruktur 110 und einem zweiten Deckel 620 an der zweiten Kontaktstruktur 120 beidseitig verschlossen ist.

Der erste und der zweite Deckel 610, 620 umfassen dazu entsprechend ausgebildete Nuten, die ausgebildet sind, um die ersten Zapfen 710 der ersten Kontaktstruktur 110 und/oder die zweiten Zapfen 720 der zweiten Kontaktstruktur 120 aufzunehmen. Da die ersten Zapfen 710 und die zweiten Zapfen 720 jeweils fest mit dem ersten Deckel 610 und dem zweiten Deckel 620 verbunden werden können (beispielsweise über eine Klebverbindung, Lötverbindung oder Schweißverbindung), kann auf diese Weise eine hohe Stabilität der Sensoranordnung erreicht werden. Der erste Deckel 610 und der zweite Deckel 620 können beispielsweise Metalldeckel sein, die eine feste mechanische Struktur bilden, um die Kräfte bzw. Drehmomente aufzunehmen, die durch die Sensoranordnung zu messen sind.

Die Raumansicht der Fig. 8B zeigt außerdem in einem zentralen Bereich entlang der axialen Achse der zylinder- oder prismenförmigen Sensoranordnung eine Öffnung 301, die beidseitig ausgebildet sein kann und sich in einem Innenbereich zu dem zuvor genannten Innenraum 300 verbreitert. Durch diese Öffnungen 301 und Innenraum 300 können weitere Instrumente oder Leitungen oder Bedienelemente durch die Sensoranordnung hindurchgeführt werden. Dies Durchgangsloch 301 kann mit einem Gewinde versehen sein, um daran den Sensor in den Kraftfluss zu montieren. Die Zuleitungen verlaufen in einem hohlen Gewindestift und können so koaxial in das Innere 300 der Schubstange geführt werden. Für die Krafteinleitung in die Sensorelemente 130 in den Diagonalstreben können die an der Ober- und Unterseite eines jeden Segmentes ausgebildete Zapfen 710, 720 genutzt werden, die in die Nuten des oberen- bzw. unteren Deckels 610, 620 greifen. Die beispielhaften Dehnungsmessstreifen 230 können wiederum innerhalb (oder auch außen) der prismenförmig angeordneten Sensoranordnung ausgebildet werden.

Alle verbleibenden Elemente können in der gleichen Weise ausgebildet sein, wie es in den zuvor gezeigten Figuren bereits beschrieben wurde.

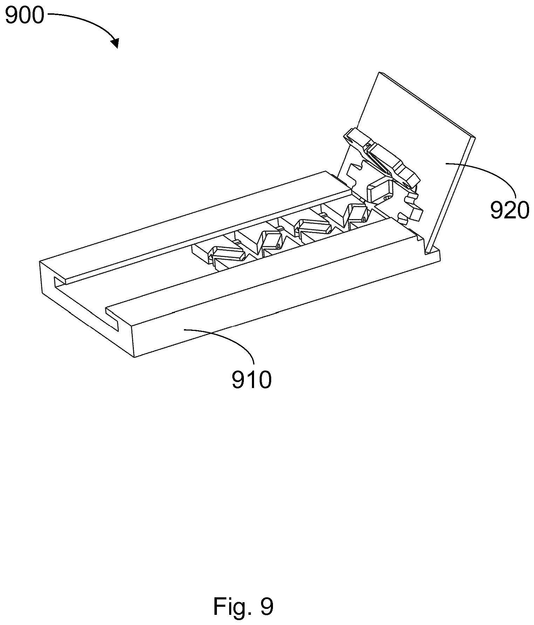

**Fig. 9** zeigt eine Vorrichtung 900, die zum Aufrollen der Sensoranordnung genutzt werden kann. Die Vorrichtung 900 umfasst einen ersten Abschnitt 910 (Führung) und einen zweiten Abschnitt 920 (abgewinkelter Abschnitt). In dem ersten Abschnitt 910 sind entsprechende Einkerbungen/Führungen (z.B. als eine T-Nut) ausgebildet, die es erlauben, die planar angeordnete Sensorstruktur, wie sie beispielsweise in der Fig. 7 gezeigt ist, einzuführen und lateral zu verschieben. Der zweite Abschnitt 920 ist beispielsweise in einem Winkel von ca. 120° relativ zu dem ersten Abschnitt 910 gekippt.

Damit wird es möglich, dass mittels eines Durchschiebens der planaren Sensorstruktur (siehe Fig. 7) die Sensorabschnitte 10, 20, ... jeweils mit einem Winkel von ca. 120° relativ zueinander abgeknickt werden, so dass sich schließlich die aufgerollte Struktur ergibt, wie sie beispielsweise in den Fig. 6, 8A oder 8B zu sehen ist. Dabei kann der zweite Abschnitt 920 insbesondere flach (parallel zum ersten Abschnitt 910) bzw. um den Winkel kippbar sein. Im Wechsel erfolgt dann ein schrittweises Voranschieben der Sensorstruktur und ein Abkippen des zweiten Abschnittes 920 relativ zum ersten Abschnitt 910 um den Winkel. Der Winkel kann beispielsweise flexibel einstellbar sein.

Aufgrund der geplanten Lage bei bestimmten Anwendungen (z.B. in einer rotierenden Schubstrebe bei einer minimal-invasiven Chirurgie) sind die Deckel 610, 620 axial und parallel zueinander auszurichten, da ansonsten die vorhandene Exzentrizität bei einer Rotation der beispielhaften Schubstrebe zu einer unerwünschten Bewegung des Endeffektors führt. Zwecks Ausrichtung einer vormontierten Baugruppe kann dazu ein möglichst passgenauer Zylinder genutzt werden.

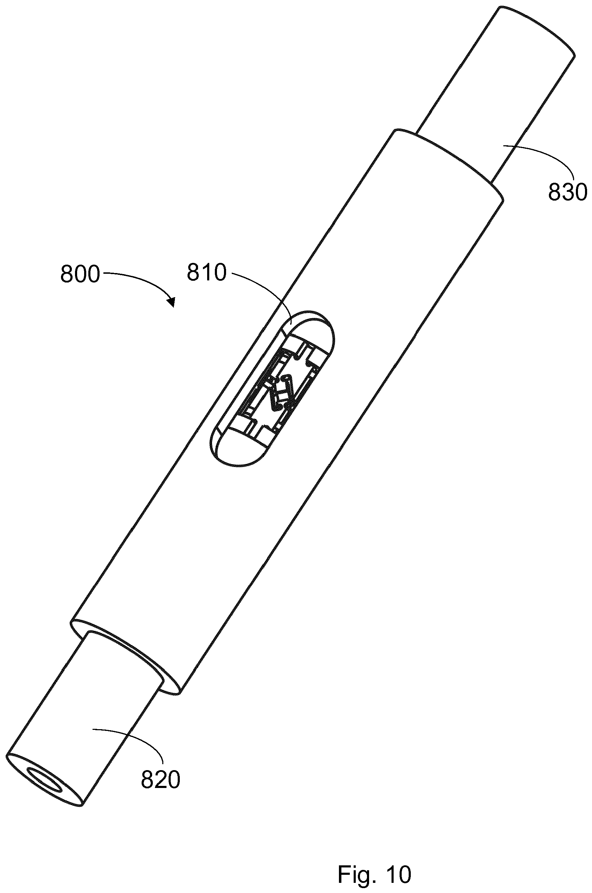

**Fig. 10** zeigt eine dazu geeignet Montagehilfe 800, die ein Ausrichten der Öffnungen 301 in dem ersten Deckel 610 und in dem zweiten Deckel 620 ermöglicht, so dass eine Verdrehung des ersten Deckels 610 um die axiale Achse zu einer Drehung des zweiten Deckels 620 um eine gleiche axiale Achse führt, und zwar ohne einen seitlichen Versatz oder Exzentrizität.

Die aufgerollte Sensorstruktur, wie sie in der Fig. 6 gezeigt ist, kann dort eingeführt werden, wobei die Deckel 610, 620 bereits auf die aufgerollte Sensorstruktur aufgesetzt werden können (siehe Fig. 8A, 8B). Optional können die Deckel 610, 620 jedoch auch anschließend eingeführt werden, d.h. von einer Seite der Montagehilfe 800 wird der erste Deckel 610 und von der anderen Seite wird der zweite Deckel 620 eingeführt. Um sicherzustellen, dass die Zapfen 710 der ersten Kontaktstruktur 110 sich in die Nuten des ersten Deckels 610 und die Zapfen 720 der zweiten Kontaktstruktur 120 in die entsprechenden Nuten des zweiten Deckels 620 hineinbewegen, kann das gezeigte Fenster 810 (z.B. als ein Langloch ausgebildet) genutzt werden, um die Sensoranordnung entsprechend zu drehen.

Unter Ausübung einer Kraft auf die Deckel 610, 620 werden diese auf die aufgerollte Struktur fest aufgedrückt. Die Kraft kann beispielsweise unter Nutzung von Stempeln (oder Zylinder) 820, 830 ausgeübt werden, die beidseitig in die Montagehilfe 800 eingeführt werden und einen Formschluss zwischen den Bauteilen herstellen können.

Die so ausgerichteten Komponenten können anschließend durch Kleben zunächst miteinander verbunden werden. Für diesen Vorgang kann das Langloch 810 in dem Hohlzylinder genutzt werden, über das die Fügestellen zugänglich sind.

Die Montagehilfe 800 stellt aufgrund der zylindrischen Anordnung sicher, dass die jeweiligen axialen Drehrichtungen aneinander ausgerichtet sind und die Sensoranordnung beim Drehen keine Unwucht oder Exzentrizität aufweist (beide Deckel drehen sich um die gleiche Drehachse).

Nach dem Verkleben kann die Sensoranordnung aus der Montagehilfe 800 entnommen werden. Ein abschließender Fügeschritt kann ein stirnseitiges Verschweißen der Zapfen 710, 720 mit den Deckeln 610, 620 umfassen. Hierfür kann zum Beispiel ein Mikrolaserschweißen genutzt werden. Um die Schweißnaht zu verbergen, kann die Länge der Zapfen 710, 720 kürzer als die Dicke der Deckel 610, 620 gewählt werden.

Die Verwendung der Zapfenkonstruktion erleichtert die Erstmontage der Baugruppe und sorgt für eine räumliche (und damit thermische) Trennung zwischen den auf der Innenseite sitzenden hitzempfindlichen Komponenten und der Schweißstelle.

Weitere Vorteile von Ausführungsbeispielen können wie folgt zusammengefasst werden:
Gegenüber den bekannten Sensoren wird die Struktur des Verformungskörpers geändert, so dass dieser zunächst mittels planar auszuführenden Prozessen gefertigt werden kann. Diese planar auszuführenden Prozesse umfassen beispielsweise eine Mikrozerspanung auf der Vorderseite und/oder der Rückseite und anschließend einen beispielhaften Schneidprozess (z.B. Laserschneiden). Auf die so entstandene ebene Struktur können im Anschluss die Dehnungsmesselemente 230 aufgebracht und angeschlossen werden. Es ist dabei vom besonderen Vorteil, dass der gesamte Aufbau und die Verbindungstechnik in planaren Fertigungsschritten mit etablierten Technologien (wie beispielsweise Drahtbonden) automatisiert erfolgen kann. Der Kraftsensor (Sensoranordnung) entsteht schließlich durch das Aufrollen des mit Dehnungsmessstreifen bestückten Verformungskörpers, wodurch die finale Hexapodstruktur entsteht.

Um die vorteilhafte planare Fertigung ausführen zu können, werden die passiven Kugelgelenke des Hexapoden der bekannten Strukturen durch Festkörpergelenke ersetzt. Diese Festkörpergelenke 111, 112, ... können beispielsweise ein ausreichend duktiles Material aufweisen, welches die Gelenkfunktionalität bereitstellt. Die längenveränderlichen Streben (Sensorelemente 130), die durch die Festkörpergelenke 111, 112, ... gehalten werden, können durch die gezeigte Brückenstruktur gebildet werden. Durch diese Brückenstruktur wird erreicht, dass nicht nur der längenveränderliche aktive Freiheitsgrad erfassbar ist, sondern dass die entstehende Dehnung vergrößert wird (durch den genannten Hebeleffekt, siehe Fig. 4 und Fig. 5). Dies ermöglicht eine erhöhte Sensitivität der gebildeten Sensoranordnung.

Weitere Vorteile von Ausführungsbeispielen können wie folgt stichpunktartig zusammengefasst werden:
- Die planare Fertigung ermöglicht eine Fertigung in hohen Stückzahlen zu geringen Kosten. Beispielsweise kann der Verformungskörper mittels einer Mikrozerspanung, einer Galvanoabformung, einem Mikrospritzprozess oder einem 3D-Druck hergestellt werden.
- Für die Mikrozerspanung ergeben sich Vorteile in Bezug auf die für die Bearbeitung notwendige Maschine, die bei dem Fertigungsprozess gemäß Ausführungsbeispielen nur in drei Achsen beweglich zu sein braucht, anstatt von fünf Achsen, wie es bei konventionellen Sensoranordnungen notwendig ist.
- Außerdem können mehrere Frästeile auf einer beispielhaften Stahltafel gefertigt werden (Batch-Prozess), wodurch ebenfalls die Rüstzeit verkürzt werden kann.

- Ferner können für die Aufbautechnik und die Verbindungstechnik etablierte Verfahren wie beispielsweise das Drahtbonden zur Kontaktierung von der Messelemente oder der Lithographie zur Strukturierung der Leiterbahnen verwendet werden.
- Ebenso kann die Miniaturisierung weiter voran gebracht werden, da die Bearbeitung chargenweise erfolgen kann und der Verformungskörper als Verbund gehandhabt werden kann.
- Die Fertigungsschritte sind außerdem automatisierbar, so dass die Miniaturisierung nur durch die Präzision der verwendeten Maschine und deren Technologie begrenzt wird.
- Der Kraftsensor bzw. die Sensoranordnung entsteht durch ein einfaches Aufrollen, so dass sich in der Mitte ein Loch ergibt, durch welches für einen beispielhaften Chirurgieroboter weitere Elemente wie beispielsweise eine Schubstange oder andere Zuleitungen, die für das Öffnen und Schließen eines Endeffektors verwendet werden, geführt werden können.
- Schließlich nutzt der Sensor den gegebenen Bauraum (Mantelfläche der zylindrischen Instrumente) optimal aus, was bei den bekannten Sensoren für mehrachsige Messungen nicht der Fall ist (wie beispielsweise bei einer Sonnenradstruktur). Gemäß Ausführungsbeispielen sind alle Stege in einer Ebene oder Kreisscheibe angeordnet.

### Bezugszeichenliste

- 10,20,...60: Sensorabschnitte
- 101: Masse
- 110: erste Kontaktstruktur
- 111,121: Gelenke
- 115: Verbindungsstelle
- 120: zweite Kontaktstruktur
- 130,131,...: Sensorelemente
- 210: erste Verbindungsabschnitte
- 211, ... 216, 221, ... 226: Kontaktpads
- 219: erste Kammstruktur
- 220: zweite Verbindungsabschnitte
- 229: zweite Kammstruktur
- 230, 231,: Dehnungsmesselemente/Dehnungsmessstreifen
- 300: Innenraum
- 301: Öffnungen der Deckel
- 330: zusätzliche/s Sensorelement(e)
- 331: Ausnehmung
- 332: abgedünnter Abschnitt einer Brückenstruk-tur/Verbindungsabschnitt
- 610,620: Deckel
- 710,720: Zapfen
- 800: Montagehilfe
- 810: Fenster
- 820,830: Stempel/Zylinder
- 900: Aufrollvorrichtung
- 910: Führung für planare Sensorstruktur
- 920: abgewickelter Abschnitt der Aufrollvorrichtung

## Patentansprüche

1. Sensoranordnung zur Messung von zumindest einer Komponente einer Kraft oder eines Drehmomentes, mit folgenden Merkmalen:
einer ersten Kontaktstruktur (110) und einer zweiten Kontaktstruktur (120), zwischen denen die zumindest eine Komponente der Kraft oder des Drehmomentes zu messen ist,
**gekennzeichnet durch**
mehrere Sensorelemente (130), die jeweils über ein erstes Gelenk (111, 112, ...) mit der ersten Kontaktstruktur (110) und über ein zweites Gelenk (121, 122, ...) mit der zweiten Kontaktstruktur (120) verbunden und ausgebildet sind, die Komponente der Kraft oder des Drehmomentes zwischen der ersten Kontaktstruktur (110) und der zweiten Kontaktstruktur (120) zu messen,
wobei die erste Kontaktstruktur (110), die zweite Kontaktstruktur (120) und die mehreren Sensorelemente (130) eine aufgerollte Sensorstruktur bilden, die sich mantelförmig oder spiralförmig entlang einer Oberfläche der Sensoranordnung erstreckt.

2. Sensoranordnung nach Anspruch 1, wobei die mehreren Sensorelemente (130) drei Sensorelemente sind, die eine Tripodstruktur definieren, oder sechs Sensorelemente (131, ... , 136) sind, die eine hexagonale Struktur definieren, und derart relativ zu der ersten Kontaktstruktur (110) und der zweiten Kontaktstruktur (120) geneigt angeordnet sind, um dadurch drei verschiedene Kraftkomponenten und/oder drei verschiedene Drehmomentkomponenten unabhängig voneinander zu messen.

3. Sensoranordnung nach Anspruch 1 oder Anspruch 2, wobei die mehreren Sensorelemente (130) jeweils eine Brückenstruktur mit einem abgedünnten Abschnitt (332) und zumindest einem Dehnungsmesselement (231) auf dem abgedünnten Abschnitt (332) umfassen, um eine Dehnung des abgedünnten Abschnittes (332) als Folge der Einwirkung der Kraft oder des Drehmomentes zu messen.

4. Sensoranordnung nach Anspruch 3,
wobei die Brückenstruktur ein U-förmiges Querschnittsprofil mit zwei gegenüberliegenden Abschnitten aufweist, zwischen denen eine Ausnehmung (331) ausgebildet ist und die mit einem Verbindungsabschnitt als abgedünnten Abschnitt (332) überbrückt sind,
und wobei die erste Kontaktstruktur (110) und die zweite Kontaktstruktur (120) an den zwei gegenüberliegenden Abschnitten koppeln und das Dehnungsmesselement (231) auf dem Verbindungsabschnitt (332) ausgebildet ist.

5. Sensoranordnung nach einem der vorhergehenden Ansprüche, wobei das erste Gelenk (111) und das zweite Gelenk (121) jeweils ein Festkörpergelenk ist, wobei das Festkörpergelenk eine verringerte, insbesondere quadratische oder runde, Querschnittsfläche aufweist.

6. Sensoranordnung nach einem der vorhergehenden Ansprüche, die weiter Folgendes umfasst:
einen ersten Deckel (610) und einen zweiten Deckel (620), wobei der erste Deckel (610) an der ersten Kontaktstruktur (110) befestigt ist und der zweite Deckel an der zweiten Kontaktstruktur (120) befestigt ist und Mittel zur Krafteinleitung oder Kraftausleitung umfassen.

7. Sensoranordnung nach Anspruch 6, wobei die Sensoranordnung ausgebildet ist zur Kraftübertragung auf ein Werkzeug,
und wobei der erste Deckel (610) und der zweite Deckel (620) eine axiale Achse definieren, um die die mantelförmige Sensorstruktur angeordnet ist, und der erste Deckel (610) und der zweite Deckel (620) jeweils eine Öffnung (301) aufweisen, durch die die axiale Achse verläuft,
und wobei die aufgerollte Sensorstruktur einen Innenraum (300) definiert, um ein Verlegen von optischen und/oder elektrischen Leitungen und/oder von Elementen zur Bedienung des Werkzeuges entlang der axialen Achse durch die Sensoranordnung hindurch zu ermöglichen.

8. Sensoranordnung nach Anspruch 7,
wobei die erste Kontaktstruktur (110) und die zweite Kontaktstruktur (120) jeweils eine Vielzahl von Segmenten umfassen und jeweils ein Sensorelement (130) zwischen zwei entsprechenden Segmenten ausgebildet ist, um eine Vielzahl von Sensorabschnitten (10, 20, ...) zu bilden, die Segmente umfassen jeweils einen Zapfen (710, 720), der sich von dem Sensormodul (130) weg erstreckt,
und wobei der erste Deckel (610) und der zweite Deckel (620) jeweils eine Vielzahl von Nuten aufweisen, die derart angeordnet sind, dass die Zapfen (710, 720) der ersten und zweiten Kontaktstruktur (110, 120) in die Nuten einführbar sind.

9. Sensoranordnung nach Anspruch 8,
die außerdem zusätzliche Sensorelemente (330) auf den Segmenten der ersten Kontaktstruktur (110) oder auf den Segmenten der zweiten Kontaktstruktur (120) umfasst,
wobei jeweils ein Sensorelement (130) mit einem der zusätzlichen Sensorelemente (330) zu einer Halbbrückenschaltung verschaltet ist.

10. Sensoranordnung nach einem der vorhergehenden Ansprüchen,
wobei die erste Kontaktstruktur (110) eine erste Kammstruktur (219) und die zweite Kontaktstruktur (120) eine zweite Kammstruktur (229) aufweisen, wobei die feste Verbindung zumindest eine der folgenden Verbindungen umfasst: eine Klebverbindung, eine Lötverbindung, eine Schweißverbindung, insbesondere unter Nutzung eines Lasers.

11. Verwendung der Sensoranordnung nach einem der Ansprüche 1 bis 10 als einen Kraft-Momentensensor für eine minimalinvasive Chirurgie.

12. Verfahren zur Herstellung eines Sensors zur Messung von zumindest einer Kraft und eines Drehmomentes, **gekennzeichnet durch** die folgenden Schritte:
Bereitstellen einer planaren Sensorstruktur mit einer ersten Kontaktstruktur (110) und einer zweiten Kontaktstruktur (120) mit dazwischen gelenkförmig verbundenen mehreren Sensorelementen (130),
Aufrollen der planaren Sensorstruktur, so dass die erste Kontaktstruktur (110) und die zweite Kontaktstruktur (120) und die mehreren Sensorelemente (130) sich mantelförmig um eine axiale Achse herum erstrecken.

13. Verfahren nach Anspruch 12, wobei das Bereitstellen der planaren Sensorstruktur folgende Schritte umfasst:
Bereitstellen eines biegsamen Grundkörpers;
Strukturieren des biegsamen Grundkörpers, um die erste Kontaktstruktur (110) und die zweite Kontaktstruktur (120) zu bilden, die durch Brückenelemente miteinander verbunden sind; und
Ausbilden von zumindest jeweils einem Dehnungsmesselement (231) auf den Brückenelementen.

14. Verfahren nach Anspruch 12 oder Anspruch 13, wobei der Schritt des Aufrollens weiter Folgendes umfasst:
Einführen der planaren Sensorstruktur in eine Aufrollvorrichtung (900), die eine Führung (910) und einen abgewinkelten Abschnitt (920) umfasst, wobei die Führung (910) ausgebildet ist, um die planare Sensorstruktur aufzunehmen; und
Bewegen der planaren Sensorstruktur hin zu dem abgewinkelten Abschnitt (920), wodurch die planare Sensorstruktur abschnittsweise verbogen und die mantelförmige Struktur erzeugt wird.

15. Verfahren nach einem der Ansprüche 12 bis 14, das weiter Folgendes umfasst:
Aufsetzen eines ersten Deckels (610) und eines zweiten Deckels (620) auf gegenüberliegenden Seiten der aufgerollten Sensorstruktur;
Einführen der aufgerollten Sensorstruktur in einer zylinder- oder prismenförmigen Montagehilfe (800); und
Ausüben eines Druckes auf die gegenüberliegenden Deckel (610, 620), um die mantelförmig angeordnete Sensorstruktur axialsymmetrisch auszurichten.

## Claims

1. A sensor arrangement for measuring at least one component of a force or torque, comprising the following features:
a first contact structure (110) and a second contact structure (120) between which the at least one component of the force or torque is to be measured,
**characterized by**
a plurality of sensor elements (130), each of which is connected to the first contact structure (110) via a first joint (111, 112, ...) and to the second contact structure (120) via a second joint (121, 122, ...) and which are designed to measure the component of the force or torque between the first contact structure (110) and the second contact structure (120),
the first contact structure (110), the second contact structure (120) and the plurality of sensor elements (130) forming a coiled sensor structure which extends along a surface of the sensor arrangement in the form of a jacket or spiral.

2. The sensor arrangement according to claim 1, wherein the plurality of sensor elements (130) are three sensor elements which define a tripod structure or six sensor elements (131, ..., 136) which define a hexagonal structure and are arranged so as to be inclined relative to the first contact structure (110) and the second contact structure (120) so as to thereby measure three different force components and/or three different torque components independently.

3. The sensor arrangement according to claim 1 or claim 2, wherein the plurality of sensor elements (130) each comprise a bridge structure having a thinned portion (332) and at least one strain measuring element (231) on the thinned portion (332) in order to measure the strain of the thinned portion (332) as a result of the application of the force or torque.

4. The sensor arrangement according to claim 3,
wherein the bridge structure has a U-shaped cross-sectional profile with two opposing portions between which a recess (331) is formed and which are bridged by a connecting portion as the thinned portion (332),
and wherein the first contact structure (110) and the second contact structure (120) couple at the two opposing portions and the strain measuring element (231) is formed on the connecting portion (332).

5. The sensor arrangement according to any of the preceding claims, wherein the first joint (111) and the second joint (121) is in each case a flexure joint, wherein the flexure joint has a reduced, in particular square or round, cross-sectional area.

6. The sensor arrangement according to any of the preceding claims, further comprising the following:
a first cover (610) and a second cover (620), wherein the first cover (610) is fastened to the first contact structure (110) and the second cover is fastened to the second contact structure (120) and said covers comprises means for introducing or dissipating force.

7. The sensor arrangement according to claim 6, wherein the sensor arrangement is designed for transmitting force to a tool,
and wherein the first cover (610) and the second cover (620) define an axial axis about which the jacket-like sensor structure is arranged, and the first cover (610) and the second cover (620) each have an opening (301) through which the axial axis extends,
and wherein the coiled sensor structure defines an interior space (300) to allow routing of optical and/or electrical lines and/or of elements for operating the tool along the axial axis through the sensor arrangement.

8. The sensor arrangement according to claim 7,
wherein the first contact structure (110) and the second contact structure (120) each comprise a large number of segments and one sensor element (130) is formed in each case between two corresponding segments to form a large number of sensor portions (10, 20,...), and the segments each comprise a pin (710, 720) extending away from the sensor module (130),
and wherein the first cover (610) and the second cover (620) each have a large number of grooves which are arranged such that the pins (710, 720) of the first and the second contact structure (110, 120) can be inserted into the grooves.

9. The sensor arrangement according to claim 8,
which also comprises additional sensor elements (330) on the segments of the first contact structure (110) or on the segments of the second contact structure (120),
wherein in each case one sensor element (130) is connected to one of the additional sensor elements (330) to form a half-bridge circuit.

10. The sensor arrangement according to any of the preceding claims,
wherein the first contact structure (110) has a first comb structure (219) and the second contact structure (120) has a second comb structure (229), wherein the rigid connection comprises at least one of the following connections: an adhesive connection, a soldered connection, or a welded connection, in particular using a laser.

11. A use of the sensor arrangement according to any of claims 1 to 10 as a force-torque sensor for minimally invasive surgery.

12. A method for producing a sensor for measuring at least one force and one torque, **characterized by** the following steps:
providing a planar sensor structure having a first contact structure (110) and a second contact structure (120) with a plurality of sensor elements (130) hinged together therebetween,
coiling up the planar sensor structure such that the first contact structure (110) and the second contact structure (120) and the plurality of sensor elements (130) extend in the form of a jacket about an axial axis.

13. The method according to claim 12, wherein providing the planar sensor structure comprises the following steps:
providing a flexible main body;
structuring the flexible main body to form the first contact structure (110) and the second contact structure (120) which are interconnected by bridge elements; and
forming at least one strain measuring element (231) on each of the bridge elements.

14. The method according to claim 12 or claim 13, wherein the coiling step further comprises the following:
inserting the planar sensor structure into a coiling device (900) that comprises a guide (910) and an angled portion (920), wherein the guide (910) is designed to receive the planar sensor structure; and
moving the planar sensor structure toward the angled portion (920), thereby bending the planar sensor structure in portions and creating the jacket-like structure.

15. The method according to any of claims 12 to 14, further comprising the following:
placing a first cover (610) and a second cover (620) on opposite sides of the coiled sensor structure;
inserting the coiled sensor structure into a cylindrical or prism-shaped assembly aid (800); and
exerting pressure on the opposing covers (610, 620) in order to axially symmetrically align the sensor structure arranged in the form of a jacket.

## Revendications

1. Agencement de capteurs permettant de mesurer au moins une composante d'une force ou d'un couple, comportant les caractéristiques suivantes :
une première structure de contact (110) et une seconde structure de contact (120), entre lesquelles l'au moins une composante de la force ou du couple doit être mesurée,
**caractérisé par**
plusieurs éléments de capteurs (130) qui sont respectivement reliés à la première structure de contact (110) par l'intermédiaire d'une première articulation (111, 112, ...) et à la seconde structure de contact (120) par l'intermédiaire d'une seconde articulation (121, 122, ...) et sont configurés pour mesurer la composante de la force ou du couple entre la première structure de contact (110) et la seconde structure de contact (120),
la première structure de contact (110), la seconde structure de contact (120) et les plusieurs éléments de capteurs (130) forment une structure de capteur enroulée qui s'étend le long d'une surface de l'agencement de capteurs sous la forme d'une coque ou d'une spirale.

2. Agencement de capteurs selon la revendication 1, dans lequel la pluralité d'éléments de capteurs (130) sont trois éléments de capteurs définissant une structure tripode ou six éléments de capteurs (131, ..., 136) définissant une structure hexagonale et sont ainsi disposés inclinés par rapport à la première structure de contact (110) et à la seconde structure de contact (120) afin de mesurer ainsi trois composantes de force différentes et/ou trois composantes de couple différentes indépendamment les uns des autres.

3. Agencement de capteurs selon la revendication 1 ou la revendication 2, dans lequel les plusieurs éléments de capteurs (130) comprennent respectivement une structure de pont comportant une section amincie (332) et au moins un élément de mesure de contrainte (231) sur la section amincie (332) afin de mesurer une contrainte de la section amincie (332) résultant de l'action de la force ou du couple.

4. Agencement de capteurs selon la revendication 3,
dans lequel la structure de pont présente un profil de section transversale en forme de U comportant deux sections opposées, entre lesquelles un évidement (331) est formé, et qui sont pontées avec une section de liaison en tant que section amincie (332),
et dans lequel la première structure de contact (110) et la seconde structure de contact (120) se couplent au niveau des deux sections opposées et l'élément de mesure de contrainte (231) est formé sur la section de liaison (332).

5. Agencement de capteurs selon l'une des revendications précédentes, dans lequel la première articulation (111) et la seconde articulation (121) sont respectivement une articulation à corps solide, l'articulation à corps solide présentant une surface de section transversale réduite, en particulier carrée ou ronde.

6. Agencement de capteurs selon l'une des revendications précédentes, comprenant en outre :
un premier couvercle (610) et un second couvercle (620), le premier couvercle (610) étant fixé à la première structure de contact (110) et le second couvercle étant fixé à la seconde structure de contact (120) et comprennent des moyens permettant d'introduire ou de dissiper une force.

7. Agencement de capteurs selon la revendication 6, dans lequel l'agencement de capteurs est configuré pour transmettre une force à un outil,
et dans lequel le premier couvercle (610) et le second couvercle (620) définissent un axe axial autour duquel la structure de capteur en forme de coque est disposée, et le premier couvercle (610) et le second couvercle (620) présentent respectivement une ouverture (301) par laquelle passe l'axe axial,
et dans lequel la structure de capteur enroulée définit un espace intérieur (300) pour permettre le passage de conducteurs optiques et/ou électriques et/ou d'éléments permettant de faire fonctionner l'outil le long de l'axe axial à travers l'agencement de capteurs.

8. Agencement de capteurs selon la revendication 7,
dans lequel la première structure de contact (110) et la seconde structure de contact (120) comprennent respectivement une pluralité de segments et un élément de capteur (130) est respectivement formé entre deux segments correspondants afin de former une pluralité de sections de capteurs (10, 20, ...), les segments comportent respectivement une cheville (710, 720) s'étendant à l'opposé du module de capteur (130),
et dans lequel le premier couvercle (610) et le second couvercle (620) présentent respectivement une pluralité de rainures qui sont disposées de telle sorte que les chevilles (710, 720) de la première et de la seconde structures de contact (110, 120) peuvent être insérées dans les rainures.

9. Agencement de capteurs selon la revendication 8,
lequel comprend également des éléments de capteurs supplémentaires (330) sur les segments de la première structure de contact (110) ou sur les segments de la seconde structure de contact (120),
un élément de capteur (130) étant respectivement connecté à l'un des éléments de capteurs supplémentaires (330) pour former une connexion en demi-pont.

10. Agencement de capteurs selon l'une des revendications précédentes,
dans lequel la première structure de contact (110) présente une première structure en peigne (219) et la seconde structure de contact (120) présente une seconde structure en peigne (229), la liaison fixe comprenant au moins l'une des liaisons suivantes : une liaison par adhésif, un liaison par brasage, une liaison par soudage, en particulier par l'utilisation d'un laser.

11. Utilisation de l'agencement de capteurs selon l'une des revendications 1 à 10 en tant que capteur force-couple pour la chirurgie mini-invasive.

12. Procédé de réalisation d'un capteur permettant de mesurer au moins une force et un couple, **caractérisé par** les étapes suivantes :
fourniture d'une structure de capteur planaire comportant une première structure de contact (110) et une seconde structure de contact (120) avec plusieurs éléments de capteurs (130) reliés de manière articulée entre eux,
enroulement de la structure de capteur planaire de sorte que la première structure de contact (110) et la seconde structure de contact (120) et les plusieurs éléments de capteurs (130) s'étendent sous la forme d'une coque autour d'un axe axial.

13. Procédé selon la revendication 12, dans lequel la fourniture de la structure de capteur planaire comprend les étapes suivantes :
fourniture d'un corps de base flexible ;
façonnage du corps de base flexible afin de former la première structure de contact (110) et la seconde structure de contact (120) qui sont reliées l'une à l'autre par des éléments de pont ; et
formation d'au moins un élément de mesure de contrainte (231) sur les éléments de pont, respectivement.

14. Procédé selon la revendication 12 ou la revendication 13, dans lequel l'étape d'enroulement comprend en outre :
l'insertion de la structure de capteur planaire dans un dispositif d'enroulement (900) qui comprend un guide (910) et une section inclinée (920), le guide (910) étant conçu pour recevoir la structure de capteur planaire ; et
le déplacement de la structure de capteur planaire vers la section inclinée (920), moyennant quoi la structure de capteur planaire est courbée au moins dans certaines sections et la structure en forme de coque est créée.

15. Procédé selon l'une des revendications 12 à 14, comprenant en outre :
le fait de placer un premier couvercle (610) et un second couvercle (620) sur des côtés opposés de la structure de capteur enroulée ;
l'insertion de la structure de capteur enroulée dans une aide au montage en forme de cylindre ou de prisme (800) ; et
l'application d'une pression sur les couvercles opposés (610, 620) afin d'aligner par symétrie axiale la structure de capteur disposée sous la forme d'une coque.
